# EUROPEAN PATENT APPLICATION

(11) **EP 4 353 192 A1**
(43) Date of publication of application: **17.04.2024**
(21) Application number: 23201620.4
(22) Date of filing: 04.10.2023
(51) Int. Cl.: A61F 2/24

(54) **VALVE PROSTHESIS HAVING DEPTH OF IMPLANT AND CLOCKING MARKERS**

(30) Priority: 11.10.2022 US 202263415052 P; 20.09.2023 US 202318470663
(71) Applicant: Medtronic Vascular Inc., Santa Rosa, CA 95403 (US)
(72) Inventor: Javani, Shahnaz, Santa Rosa, 95403 (US); Nigade, Anish S., Santa Rosa, 95403 (US)
(74) Representative: Zimmermann & Partner Patentanwälte mbB

(57) **Abstract**

The techniques of this disclosure generally relate to a valve prosthesis having a stent structure having an inflow portion. The inflow portion includes crowns and rows of nodes where the crowns connect, the rows of nodes including a first row, the first row being the most proximal of the rows. The valve prosthesis further includes alignment markers at each node of the first row. The alignment markers form a ring of distinct marker points around the circumference of the stent structure, wherein each distinct marker point is an equal distance from the inflow end. The alignment markers allow for accurate depth positioning of the valve prosthesis, in a crimped or compressed state, such that the valve prosthesis can be accurately deployed.

## Description

### CROSS-REFERENCE TO RELATED APPICATIONS

This application claims the benefit of U.S. Provisional Application No. 63/415,052, filed October 11, 2022, the contents of which are incorporated by reference herein in their entirety.

### FIELD

The present technology is generally related to transcatheter heart valves.

### BACKGROUND

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

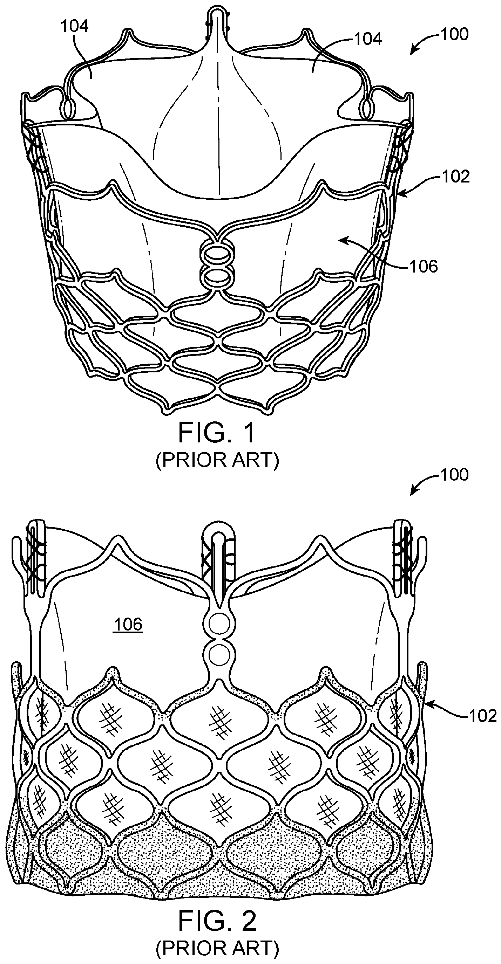

Recently, flexible prosthetic valves supported by stent structures that can be delivered percutaneously using a catheter-based delivery system have been developed for heart and venous valve replacement. FIG. 1 is a perspective view of a valve prosthesis 100 in accordance with the prior art. FIG. 2 is a side view of valve prosthesis 100 of FIG. 1 in accordance with the prior art. Referring to FIGS. 1 and 2, valve prosthesis 100 may include a balloon-expandable stent structure 102 with valve leaflets 104 attached to the interior of stent structure 102. Valve leaflets 104 may be parts of a 3D single piece prosthetic valve 106.

Valve prosthesis 100 can be reduced in diameter, by crimping onto a balloon catheter, and advanced through the venous or arterial vasculature. Once valve prosthesis 100 is positioned at the treatment site, for instance within an incompetent native valve, stent structure 102 may be expanded to hold valve prosthesis 100 firmly in place.

When designing a valve prosthesis such as valve prosthesis 100, valve-frame integration and frame mechanical performance often have competing needs or requirements. Embodiments hereof relate to an improved balloon-expandable transcatheter valve prosthesis configured to minimize tradeoffs between the above-described competing needs.

### SUMMARY

The techniques of this disclosure generally relate to a valve prosthesis having a stent structure having an inflow portion. The inflow portion includes crowns and rows of nodes where the crowns connect, the rows of nodes including a first row, the first row being the most proximal of the rows. The valve prosthesis further includes alignment markers at each node of the first row. The alignment markers form a ring of distinct marker points around the circumference of the stent structure, wherein each distinct marker point is an equal distance from an inflow end. The alignment markers allow for accurate depth positioning of the valve prosthesis, in a crimped or compressed state, such that the valve prosthesis can be accurately deployed.

In one aspect, the present disclosure provides a valve prosthesis having a stent structure having an outflow portion and an inflow portion. The outflow portion includes commissure posts and non-commissure posts. The inflow portion includes crowns and rows of node where the crowns connect, the rows of nodes including a first row, the first row being the most proximal of the rows. The valve prosthesis further includes alignment markers only at each node of the first row that is radially aligned with a commissure post. The alignment markers allow for accurate depth positioning as well as clocking of the valve prosthesis, in a crimped or compressed state, such that the valve prosthesis can be accurately deployed.

In another aspect, the present disclosure provides the stent structure with containment members, where the alignment markers are within the containment members. Strain relief features, sometimes called mousebite features, at intersections of the containment members and the crowns are provided. The strain relief features prevent stress differentials between the crowns creating uniform expansion of the valve prosthesis as well as increasing the lifespan of the valve prosthesis in situ.

In another aspect, the present disclosure provides a valve prosthesis having a stent structure having an outflow portion and an inflow portion. The outflow portion includes commissure posts and non-commissure posts. The inflow portion includes struts, crowns where the struts connect, and nodes where the crowns connect. The valve prosthesis further includes an alignment marker in a strut extending from a node of the nodes, the node being radially aligned with a commissure post.

Further disclosed herein is a valve prosthesis having a stent structure having an inflow portion, wherein the inflow portion includes crowns and rows of nodes where the crowns connect, the rows of nodes including a first row, the first row being the most proximal of the rows, wherein the valve prosthesis further includes alignment markers at each node of the first row, wherein the alignment markers form a ring of distinct marker points around the circumference of the stent structure, wherein each distinct marker point is an equal distance from the inflow end, wherein the alignment markers allow for accurate depth positioning of the valve prosthesis, in a crimped or compressed state, such that the valve prosthesis can be accurately deployed.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a valve prosthesis in accordance with the prior art.
FIG. 2 is a side view of the valve prosthesis of FIG. 1 in accordance with the prior art.
FIG. 3 is a perspective view of a valve prosthesis in an expanded configuration in accordance with one embodiment.
FIG. 4 is an outflow end view of a prosthetic valve of the valve prosthesis of FIG. 3 in accordance with one embodiment.
FIG. 5 is a perspective view of the prosthetic valve of the valve prosthesis of FIG. 3 in accordance with one embodiment.
FIG. 6 is a side view of the valve prosthesis of FIG. 3 in accordance with one embodiment.
FIG. 7 is a cutaway partial flat plan view of a stent structure of the valve prosthesis of FIGS. 3, 6 in accordance with one embodiment.
FIG. 8 is a cutaway partial flat plan view of a stent structure for use in the valve prosthesis of FIGS. 3, 6 in accordance with another embodiment.
FIG. 9 is an enlarged view of a region IX of the stent structure of FIG. 8 in accordance with one embodiment.
FIG. 10 is a cutaway partial flat plan view of a stent structure for use in the valve prosthesis of FIGS. 3, 6 in accordance with another embodiment.
FIG. 11 is a cutaway partial flat plan view of a stent structure for use in the valve prosthesis of FIGS. 3, 6 in accordance with another embodiment.
FIG. 12 is a cutaway partial flat plan view of a stent structure for use in the valve prosthesis of FIGS. 3, 6 in accordance with another embodiment.
FIG. 13 is a cutaway partial flat plan view of a stent structure for use in the valve prosthesis of FIGS. 3, 6 in accordance with another embodiment.

### DETAILED DESCRIPTION

Specific embodiments are now described with reference to the figures, wherein like reference numbers indicate identical or functionally similar elements. The terms "distal" and "proximal", when used in the following description to refer to a native vessel, native valve, or a device to be implanted into a native vessel or native valve, such as a heart valve prosthesis, are with reference to the direction of blood flow. Thus, "distal" and "distally", also referred to as the "outflow" or "outflow direction", respectively, refer to positions in a downstream direction with respect to the direction of blood flow. The terms "proximal" and "proximally", also referred to as the "inflow" or "inflow direction", respectively, refer to positions in an upstream direction with respect to the direction of blood flow.

Although the description is in the context of treatment of an aortic heart valve, embodiments may also be used where it is deemed useful in other valved intraluminal sites that are not in the heart. For example, embodiments may be applied to other heart valves or venous valves as well.

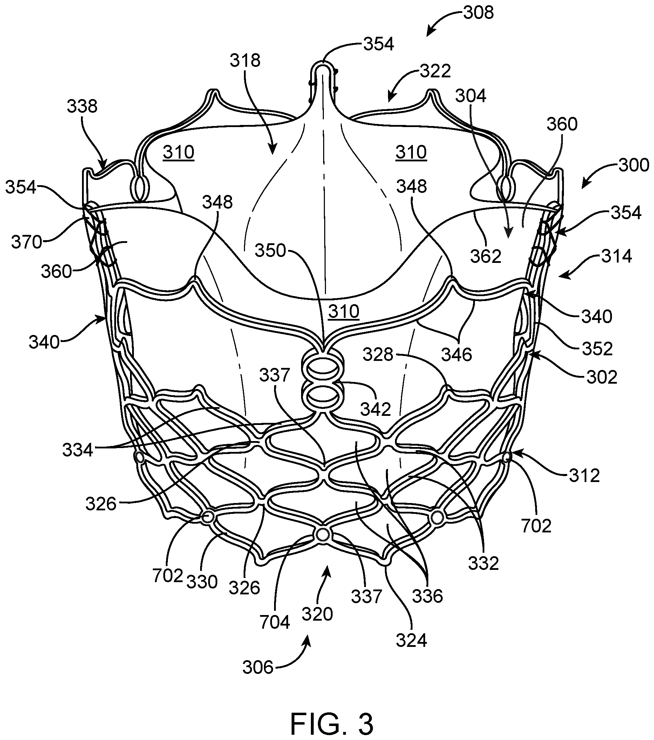

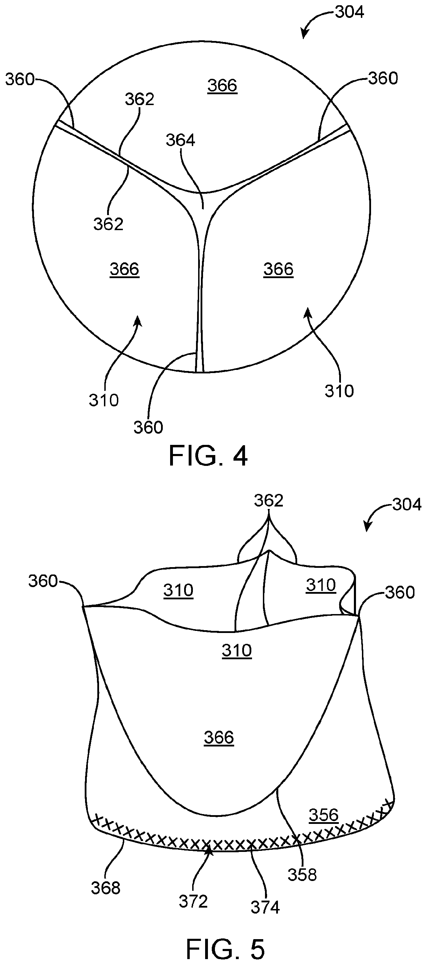

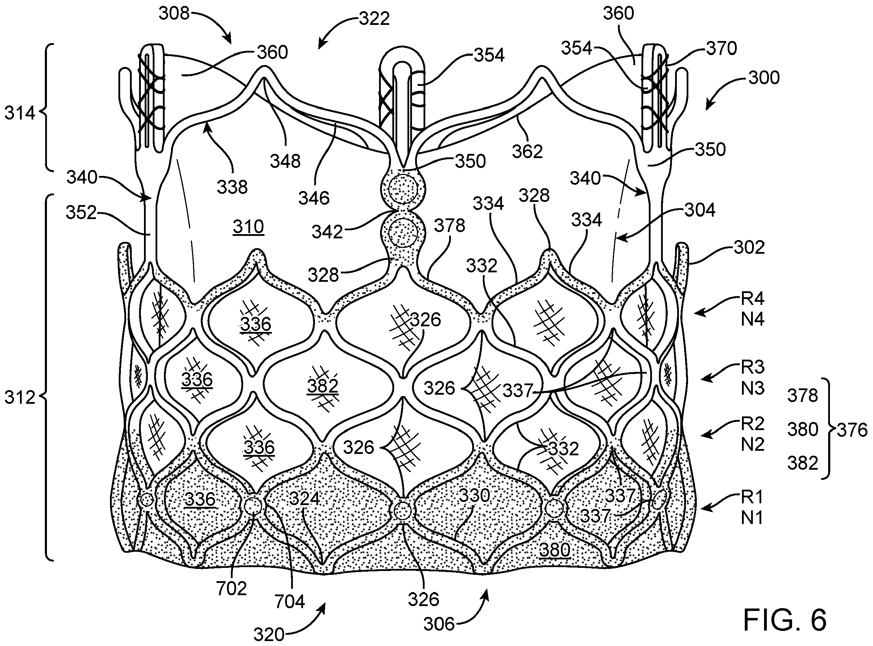

FIG. 3 is a perspective view of a transcatheter valve prosthesis 300 in an expanded configuration in accordance with one embodiment. FIG. 4 is an outflow end view of a prosthetic valve 304 of valve prosthesis 300 of FIG. 3 in accordance with one embodiment. FIG. 5 is a perspective view of prosthetic valve 304 of valve prosthesis 300 of FIG. 3 in accordance with one embodiment. FIG. 6 is a side view of valve prosthesis 300 of FIG. 3 in accordance with one embodiment. FIG. 4 illustrates prosthetic valve 304 in a closed state while FIGS. 3, 5 and 6 illustrates prosthetic valve 304 in an open state.

Referring now to FIGS. 3-6 together, valve prosthesis 300 has a radially-expandable stent structure 302, sometimes called the frame, and prosthetic valve 304. Stent structure 302 is generally tubular, and is mechanically or balloon expandable, having a crimped configuration for delivery within a vasculature and an expanded configuration for deployment within a native heart valve. When valve prosthesis 300 is deployed within the valve annulus of a native heart valve, stent structure 302 of valve prosthesis 300 is configured to be radially expanded within native valve leaflets of the patient's defective valve, to thereby retain the native valve leaflets in a permanently open state. In embodiments hereof, valve prosthesis 300 is configured for replacement of an aortic valve such that an inflow end 306, i.e., the proximal end, of valve prosthesis 300 extends into and anchors within the aortic annulus of a patient's left ventricle, while an outflow end 308, i.e., the distal end, of valve prosthesis 300 is positioned within the aortic sinuses.

Stent structure 302 of valve prosthesis 300 may be a unitary frame or scaffold that supports prosthetic valve 304 including one or more valve leaflets 310 within the interior of stent structure 302. Prosthetic valve 304 is capable of blocking flow in one direction to regulate flow there-through via valve leaflets 310 that may form a bicuspid or tricuspid replacement valve. FIG. 4 illustrates that prosthetic valve 304 has three valve leaflets 310, i.e., prosthetic valve 304 has a tricuspid leaflet configuration, although a bicuspid leaflet configuration may be used in other embodiments. More particularly, as valve prosthesis 300 is configured for placement within a native aortic valve which typically has three leaflets, prosthetic valve 304 may include three valve leaflets 310. However, valve prosthesis 300 is not required to have the same number of leaflets as the native valve. If valve prosthesis 300 is alternatively configured for placement within a native valve having two leaflets such as the mitral valve, prosthetic valve 304 may include two or three valve leaflets 310.

Stent structure 302 is balloon-expandable. As such, stent structure 302 is made from a plastically deformable material such that when expanded by a dilatation balloon, stent structure 302 maintains its radially expanded configuration. Stent structure 302 may be formed from stainless steel such as 316L or other suitable metal, such as platinum iridium, cobalt chromium alloys such as MP35N or L605, or various types of polymers or other similar materials, including the materials coated with various surface deposits to improve clinical functionality. Stent structure 302 is configured to be rigid such that it does not deflect or move when subjected to in-vivo forces, or such that deflection or movement is minimized when subjected to in-vivo forces.

Stent structure 302 includes an inflow portion 312 and an outflow portion 314. Stent structure 302 is a tubular component defining a central lumen or passageway 318, and has an inflow end 320 and an outflow end 322. When expanded, a diameter of inflow end 320 of stent structure 302 is substantially the same as a diameter of outflow end 322 of stent structure 302 in one embodiment.

Inflow portion 312 extends distally from inflow end 320 of stent structure 302. Inflow portion 312 includes inflow crowns 324, central crowns 326, outflow crowns 328, inflow struts 330, central struts 332, and outflow struts 334. Accordingly, inflow end 320 of stent structure 302 is defined by inflow crowns 324, inflow struts 330, and central crowns 326.

Inflow portion 312 includes inflow portion cells 336, sometimes called side openings, formed in rows, and more particularly, in four rows R1, R2, R3, and R4. Each row R1, R2, R3, R4 includes 12 inflow portion cells 336 in this embodiment. Inflow portion cells 336 of the proximal most row R1 are defined by inflow crowns 324, inflow struts 330, central crowns 326, and central struts 332. Inflow portion cells 336 of the next most proximal rows R2, R3 are defined by central crowns 326 and central struts 332. Inflow portion cells 336 of the distal most row R4 are defined by outflow crowns 328, outflow struts 334, central crowns 326, and central struts 332. Generally, inflow portion cells 336 are diamond-shaped openings having the same or identical shaped, sometimes are called symmetric.

Generally, a crown is defined where two struts connect and a node is defined as a region where two crowns connect. Accordingly, inflow crowns 324 are defined where inflow struts 330 connect. Outflow crowns 328 are defined where outflow struts 334 connect. Central crowns 326 are defined where inflow struts 330 connect, where outflow struts 334 connect, and where central struts 332 connect.

Central nodes 337 are defined where central crowns 326 connect. In this embodiment, there are four rows N1, N2, N3, and N4 of central nodes 337, row N1 being the most proximal row, then row N2, N3, and finally row N4 is the most distal row of central nodes 337.

Outflow portion 314 is formed proximate to outflow end 322 of stent structure 302 and between outflow end 322 and inflow portion 312. Outflow portion 314 includes an outflow crown ring 338, commissure posts 340, and non-commissure posts 342. Outflow portion 314 can be configured in a shape that forms a central lumen or passageway.

Outflow crown ring 338 includes outflow portion struts 346, superior crowns 348, and inferior crowns 350. Each outflow portion strut 346 is connected on one end to an adjacent outflow portion strut 346 at a superior crown 348 and on the opposite end to an adjacent outflow portion strut 346 at an inferior crown 350. Inferior crowns 350 are connected to either a commissure post 340 or a non-commissure post 342. More particularly, every other inferior crown 350 is connected to a commissure post 340 and every other inferior crown 350 is connected to a non-commissure post 342 in an alternating repeating arrangement.

Non-commissure posts 342, sometimes called axial frame members 342, extend longitudinally between and connect inferior crowns 350 of outflow crown ring 338 and outflow crowns 328 of inflow portion 312. In accordance with this embodiment, non-commissure posts 342 are shaped as a figure eight and can be used for clocking of valve prosthesis 300, i.e., the particular rotational alignment of valve prosthesis 300. As used herein, longitudinally is in a direction parallel with the longitudinal axis, radially is perpendicular and in a radial direction from the longitudinal axis, circumferentially is in a plane perpendicular to the longitudinal axis and in a direction along the circumference of valve prosthesis 300. Clocking refers to a particular radial orientation, i.e., a particular rotational position relative to one or more anatomical features such as an incompetent native valve. Depth of implant refers to a particular longitudinal position of valve prosthesis relative to one or more anatomical features such as an incompetent native valve.

Commissure posts 340 extend longitudinally and include axial frame members 352 and trident posts 354. Axial frame members 352 extend longitudinally between and connect inferior crowns 350 of outflow crown ring 338 and outflow crowns 328 of inflow portion 312. Trident posts 354 extend in a cantilever fashion and in the outflow or distal direction from inferior crowns 350 of outflow crown ring 338. Axial frame members 352 and trident posts 354 are parallel with one another and are segments of commissure posts 340, which are linear members.

Prosthetic valve 304 is a one piece 3D molded structure in accordance with this embodiment. Illustratively, a single cylindrical piece is molded to form prosthetic valve 304. Accordingly, although various structures of prosthetic valve 304 are discussed below, prosthetic valve 304 is integral, i.e., formed from a single piece and not a plurality of separate pieces connected together.

Prosthetic valve 304 may be made of pericardial material; however, may instead be made of another material. Natural tissue for prosthetic valve 304 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as prosthetic valve 304 include DACRON polyester commercially, other cloth materials, nylon blends, polymeric materials, and vacuum deposition nitinol fabricated materials. One polymeric material from which prosthetic valve 304 can be made is an ultra-high molecular weight polyethylene material. With certain materials, it may be desirable to coat one or both sides of prosthetic valve 304 with a material that will prevent or minimize overgrowth. It is further desirable that the material is durable and not subject to stretching, deforming, or fatigue.

Prosthetic valve 304 includes valve leaflets 310 and a valve inflow cylinder 356 proximal of valve leaflets 310. For example, valve inflow cylinder 356 is the remaining unmolded portion of the cylindrical material used to form prosthetic valve 304 and valve leaflets 310 are the molded portion.

Valve leaflets 310 are defined by cusps 358, commissures 360, and free edges 362. Adjoining pairs of valve leaflets 310 are attached to one another at their lateral ends to form commissures 360, with free edges 362 of valve leaflets 310 forming coaptation edges that meet in an area of coaptation 364. The region within cusps 358, commissures 360, and free edges 362 are sometimes referred to as a belly 366 of valve leaflets 310.

Valve inflow cylinder 356 has a cylindrical inflow end 368, sometimes called a nadir 368. Valve inflow cylinder 356 extends in the outflow direction from inflow end 368 as a cylinder to cusps 358, which form the outflow end of valve inflow cylinder 356.

Prosthetic valve 304 is disposed within and secured to at least trident posts 354 of commissure posts 340 of stent structure 302. In addition, prosthetic valve 304 may also be disposed within and secured to inflow portion 312 of stent structure 302. More particularly, commissures 360 are attached to trident posts 354, e.g., with commissure stitching 370. Further, a margin of attachment (MOA) 372 of valve inflow cylinder 356, e.g., a region directly adjacent inflow end 368, is attached to inflow portion 312, e.g., with MOA stitching 374.

Valve prosthesis 300 further includes a skirt 376, e.g., formed of graft material, which encloses or lines a portion of stent structure 302. Skirt 376 is not illustrated in FIG. 3 to allow visualization of stent structure 302. Margin of attachment 372 of valve inflow cylinder 356 is sutured or otherwise securely and sealingly attached to the interior surface of skirt 376 and to inflow portion 312, e.g., with MOA stitching 374.

Skirt 376 may enclose or line stent structure 302. Skirt 376 may be a natural or biological material such as pericardium or another membranous tissue such as intestinal submucosa. Alternatively, skirt 376 may be a low-porosity woven fabric, such as polyester, Dacron fabric, or PTFE. In one embodiment, skirt 376 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side.

In accordance with this embodiment, skirt 376 includes an outflow end outer skirt 378, and inflow end outer skirt 380, and an inner skirt 382. Outflow end outer skirt 378 is located on the outer surface of outflow crowns 328 and outflow struts 334 of inflow portion 312. Inflow end outer skirt 380 is located on the outer surface of inflow crowns 324, inflow struts 330, central crowns 326, and central struts 332. More particularly, inflow end outer skirt 380 covers first row R1 of inflow portion cells 336. Inner skirt 382 is located on the entirety of inner surface of inflow portion 312.

Delivery of valve prosthesis 300 may be accomplished via a percutaneous transfemoral approach or a transapical approach directly through the apex of the heart via a thoracotomy, or may be positioned within the desired area of the heart via different delivery methods known in the art for accessing heart valves. During delivery, valve prosthesis 300 remains compressed until it reaches a target diseased native heart valve, at which time a balloon of a delivery system is inflated in order to radially expand valve prosthesis 300 in situ. Valve prosthesis 300 is configured to be expanded within the native valve leaflets of the patient's defective valve, to thereby retain the native valve leaflets in a permanently open state. The delivery system is then removed and transcatheter valve prosthesis 300 remains deployed within the native target heart valve. Alignment markers of stent structure 302 are used for depth of implant (DOI) as well as clocking alignment during deployment of valve prosthesis 300 as set forth below in various embodiments.

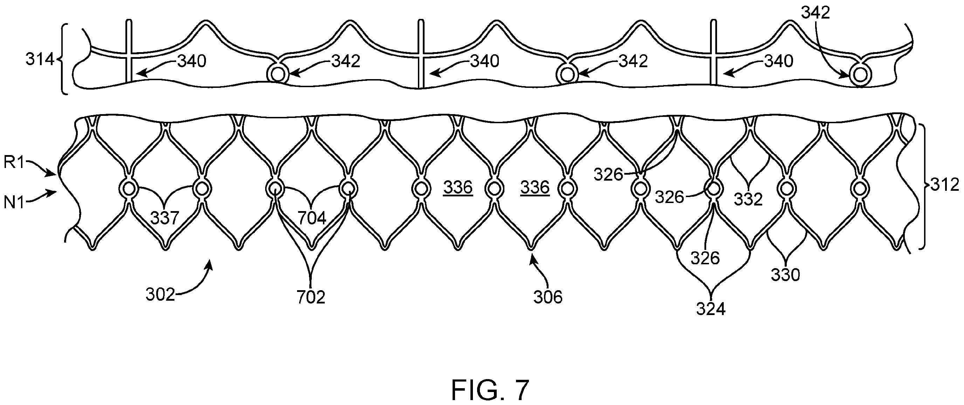

FIG. 7 is a cutaway partial flat plan view of stent structure 302 of valve prosthesis 300 of FIGS. 3, 6 in accordance with one embodiment. In FIG. 7, only first row R1 of inflow portion cells 336 and outflow portion 314 are illustrated for simplicity and to illustrate the relationship between the elements. Referring now to FIGS. 3, 6-7, to allow visualization of the depth of implant of valve prosthesis 300, and more generally to ensure the proper placement in the native anatomy of a subject, valve prosthesis 300 can include the one or more alignment markers 702 within inflow portion 312.

As discussed above, inflow portion cells 336 of the proximal most row R1 are defined by inflow crowns 324, inflow struts 330, central crowns 326, and central struts 332. In this embodiment, alignment markers 702 are positioned at each central node 337 of first row N1 of central nodes 337 and in between inflow portion cells 336 in the circumferential direction. Stated another way, alignment markers 702 are circumferentially aligned with each other around a circumference of stent structure 302.

In embodiments, alignment markers 702 can be formed in any shape to assist in the alignment of the valve prosthesis 300. In this particular embodiment, alignment markers 702 are formed having a circular shape. In other embodiments, alignment markers 702 can be formed in any other 2D or 3D shape, which has any type of 2D or 3D cross-sectional shape, such as pins, dots, ovals, spheres, triangles, cones, squares, cubes, bars, crosses, bands, rings, letters, and combination thereof.

In embodiments, alignment markers 702 include radiopaque or other material that allow alignment markers 702 to be detected and/or viewed during deployment of valve prosthesis 300. Examples of radiopaque materials include metals, e.g., stainless steel, titanium, tungsten, tantalum, gold, platinum, platinum-iridium, and/or other polymeric materials, e.g., nylon, polyurethane, silicone, pebax, PET, polyethylene, that have been mixed or compounded with compounds of barium, bismuth and/or zirconium, e.g., barium sulfate, zirconium oxide, bismuth sub-carbonate, etc.

In embodiments, alignment markers 702 are attached to stent structure 302 within a containment member 704 of stent structure 302. Containment member 704 is configured as a hollow structure or opening in stent structure 302 which can receive alignment markers 702. Alignment markers 702 can be attached to, positioned in, and/or formed in containment member 704 utilizing any type of processes and/or procedure. In an embodiment, radiopaque beads or spheres (or lines of radiopaque beads or spheres) may be press fit, swaged, interference fit, or otherwise positioned into containment member 704 to form alignment markers 702.

In an embodiment, containment member 704 is open to the interior and exterior of the stent structure 302, thereby allowing alignment markers 702 to be exposed to the interior and exterior of stent structure 302 and increasing visibility at multiple angles. In one embodiment, to account for the additional space for alignment markers 702 and containment members 704, one row of inflow portion cells 336 of inflow portion 312 are eliminated.

Containment member 704 is configured in a shape that matches a shape of alignment marker 702. For example, as illustrated in FIGS. 3, 6-7, if alignment markers 702 have a circular shape, containment member 704 is a circular cavity, e.g., a hollow ring. In one embodiment, containment members 704 are used for visualization, and alignment markers 702 are not formed.

In this embodiment, for each commissure post 340 and each non-commissure post 342, a radially aligned alignment marker 702 exists. In addition, directly between commissure post 340 and each non-commissure post 342, a radially aligned alignment marker 702 exists. More particularly, there are three commissure posts 340, three non-commissure posts 342, and 12 alignment markers 702.

In this embodiment, alignment markers 702 form a ring of distinct marker points around the circumference of stent structure 302, wherein each distinct marker point is an equal distance from inflow end 306. Alignment markers 702 allow for accurate depth positioning of valve prosthesis 300, in a crimped or compressed state, such that valve prosthesis 300 can be accurately deployed and reduce the rate of complications, e.g., reduce the incidence rate of permanent pacemaker (PPM) post-implantation.

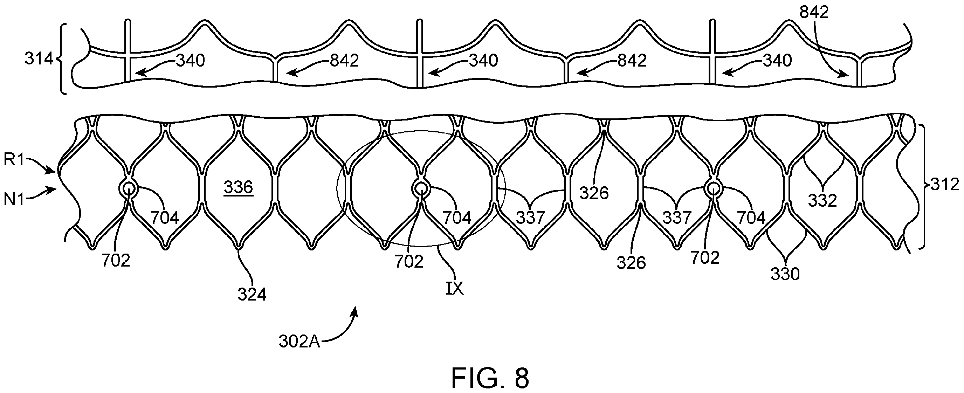

In one embodiment, stent structure 302 includes non-commissure posts 342 which are shaped as a figure 8 as discussed above. In accordance with this embodiment, alignment markers 702 are used for depth of implant (DOI), i.e., are DOI markers, whereas the figure 8 shape of non-commissure posts 342 are used for radial orientation, also called clocking, i.e., are clocking markers, of valve prosthesis 300.

While FIGS. 3, 6-7 illustrates one example of the positioning and number of alignment markers 702, one skilled in the art will realize that stent structure 302 can include any number of alignment markers 702, positioned at any location within the inflow portion 312. For example, alignment markers 702 can be positioned on inflow struts 330 or central struts 332. Likewise, for example, alignment markers 702 can be asymmetrically aligned, circumferentially, around a circumference of stent structure 302. Additionally, for example, alignment markers 702 can be positioned at different distances from inflow end 306. Various examples are provided below in reference to FIGS. 8-13 in accordance with various embodiments.

FIG. 8 is a cutaway partial flat plan view of a stent structure 302A for use in valve prosthesis 300 of FIGS. 3, 6 in accordance with one embodiment. Stent structure 302A of FIG. 8 is similar to stent structure 302 of FIGS. 3, 6-7 and only the significant differences are discussed below.

Referring now to FIG. 8, to allow for clocking as well as depth of implant, stent structure 302A includes only three alignment markers 702, sometimes called DOI and clocking markers. More specifically, each alignment marker 702 is radially aligned with a commissure post 340. In contrast to the embodiment of FIG. 7, by forming only three alignment markers 702, the radial orientation of stent structure 802 can be readily visualized. In first row N1 of central nodes 337, there are three central nodes 337 without alignment markers 702 for each central node 337 with an alignment marker 702. In other embodiment, instead of being aligned with commissure posts 340, alignment markers 702 are aligned with non-commissure posts 842. In yet another embodiment, there are more or less than three alignment markers 702, e.g., six alignment markers 702 aligned with commissure posts 340 and non-commissure posts 842.

Further, as alignment markers 702 are used for clocking, the figure 8 shaped non-commissure posts 342 as illustrated in FIGS. 3, 6-7 are unnecessary. Accordingly, in stent structure 302A of FIG. 8, non-commissure posts 842 are formed as straight longitudinal members.

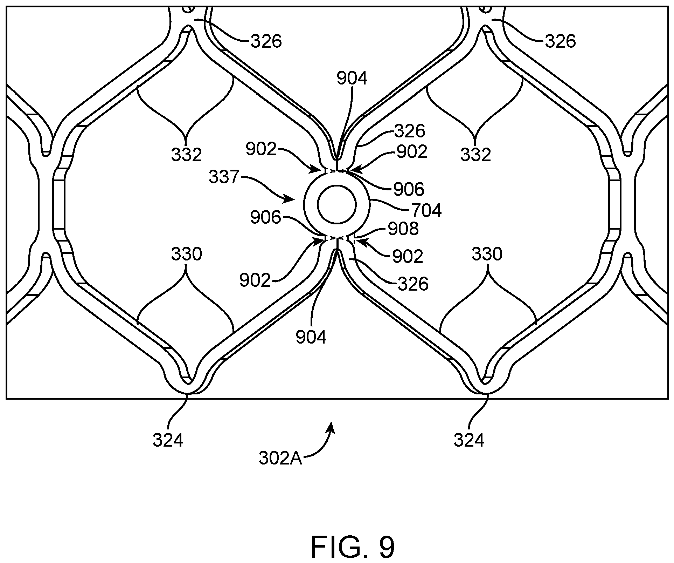

FIG. 9 is an enlarged view of a region IX of stent structure 302A of FIG. 8 in accordance with one embodiment. Paying particular attention now to FIG. 9, at the intersection of each containment member 704 and central crown 326, i.e., at central nodes 337, strain relief features 902, sometimes called mousebite features or cut-out features, are provided. Specifically, at each intersection of a containment member 704 and central crown 326, two strain relief features 902 are provided, for a total of four strain relief features 902 at the respective central node 337.

As illustrated, strain relief features 902 are cutouts or indentations that provide a thinning at each side in the circumferential direction of the intersection of a containment member 704 and a central crown 326. More particularly, the curve of central crowns 326 abut the circle of containment member 704 without any overlap of the shapes to define strain relief features 902. The shapes of the curve of central crowns 326 are illustrated in the phantom lines 904 and the shape of the circle of containment member 704 is illustrated in the phantom lines 906.

By providing strain relief features 902, a thickening of material of stent structure 302A at the intersection of a containment member 704 and a central crown 326 is avoided. Such a thickening of material is illustrated by the phantom line 908. Stated another way, strain relief features 902 maintain the thickness of central crowns 326, i.e., the profile of a central crown 326 connected to a containment member 704 matches the profile of a central crown 326 directly connected to another central crown 326.

By maintaining the thickness of central crowns 326 to be uniform, stress differentials between central crowns 326 is avoided. This, in turn, creates uniform expansion of valve prosthesis 300 as well as increases the lifespan of valve prosthesis 300 in situ.

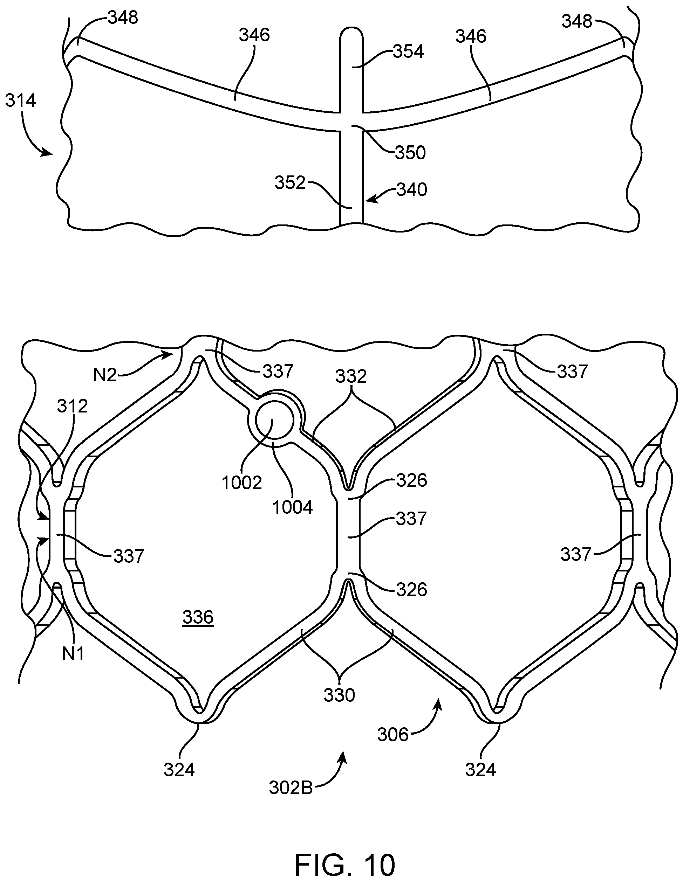

FIG. 10 is a cutaway partial flat plan view of a stent structure 302B for use in valve prosthesis 300 of FIGS. 3, 6 in accordance with one embodiment. Stent structure 302B of FIG. 10 is similar to stent structure 302A of FIG. 8 and only the significant differences are discussed below.

Referring now to FIGS. 8 and 10 together, in place of alignment markers 702, stent structure 302B includes alignment markers 1002 formed in central struts 332 of inflow portion 312. More particularly, alignment markers 1002 are formed in central struts 332 extending distally from first row N1 of central nodes 337 aligned with commissure posts 340. In other words, from a central node 337 in first row N1 of central nodes 337 that is aligned with a commissure post 340, a central strut 332 extends to a central node 337 of second row N2 of central nodes 337. In this central strut 332, an alignment marker 1002 and containment member 1004 are formed at the center of the central strut 332. Alignment marker 1002 and containment member 1004 are similar to alignment marker 702 and containment member 704 and so are not discussed in detail for clarity.

Central strut 332 having alignment marker 1002 extends to the left in the view of FIG. 10 from the radial position of the respective commissure post 340, or counterclockwise when viewed from inflow end 336. Accordingly, alignment marker 1002 is radially offset counterclockwise from the respective commissure post 340. By knowing the radial offset from the respective commissure post 340 as well as the longitudinal offset from inflow end 306, the physician can radially and longitudinally align valve prosthesis 300 during delivery.

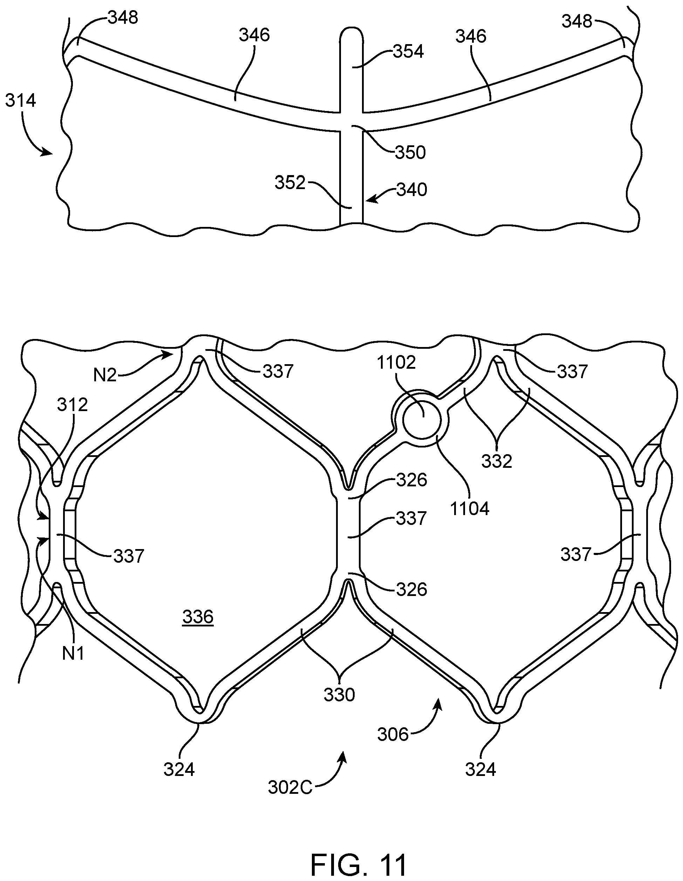

FIG. 11 is a cutaway partial flat plan view of a stent structure 302C for use in valve prosthesis 300 of FIGS. 3, 6 in accordance with one embodiment. Stent structure 302C of FIG. 11 is similar to stent structure 302A of FIG. 8 and only the significant differences are discussed below.

Referring now to FIGS. 8 and 11 together, in place of alignment markers 702, stent structure 302C includes alignment markers 1102 formed in central struts 332 of inflow portion 312. More particularly, alignment markers 1102 are formed in central struts 332 extending distally from first row N1 of central nodes 337 aligned with commissure posts 340. In other words, from a central node 337 in first row N1 of central nodes 337 that is aligned with a commissure post 340, a central strut 332 extends to a central node 337 of second row N2 of central nodes 337. In this central strut 332, alignment marker 1102 and containment member 1104 is formed at the center of the central strut 332. Alignment marker 1102 and containment member 1104 are similar to alignment marker 702 and containment member 704 and so are not discussed in detail for clarity.

Central strut 332 having alignment marker 102 extends to the right in the view of FIG. 11 from the radial position of the respective commissure post 340, or clockwise when viewed from inflow end 306. Accordingly, alignment marker 1102 is radially offset clockwise from the respective commissure post 340. By knowing the radial offset from the respective commissure post 340 as well as the longitudinal offset from inflow end 306, the physician can radially and longitudinally align valve prosthesis 300 during delivery.

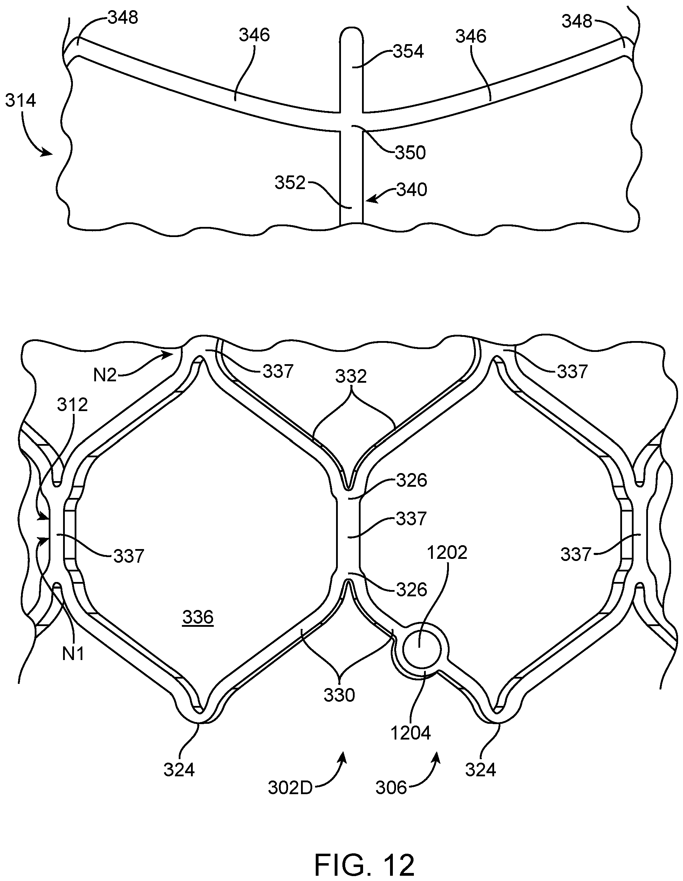

FIG. 12 is a cutaway partial flat plan view of a stent structure 302D for use in valve prosthesis 300 of FIGS. 3, 6 in accordance with one embodiment. Stent structure 302D of FIG. 12 is similar to stent structure 302A of FIG. 8 and only the significant differences are discussed below.

Referring now to FIGS. 8 and 12 together, in place of alignment markers 702, stent structure 302D includes alignment markers 1202 formed in inflow struts 330 of inflow portion 312. More particularly, alignment markers 1202 are formed in inflow struts 330 extending proximally from first row N1 of central nodes 337 aligned with commissure posts 340. In other words, from a central node 337 in first row N1 of central nodes 337 that is aligned with a commissure post 340, an inflow struts 330 extends to an inflow crown 324. In this inflow strut 330, an alignment marker 1202 and containment member 1204 are formed at the center of the inflow strut 330. Alignment marker 1202 and containment member 1204 are similar to alignment marker 702 and containment member 704 and so are not discussed in detail for clarity.

Inflow strut 330 having alignment marker 1202 extends to the right in the view of FIG. 12 from the radial position of the respective commissure post 340, or clockwise when viewed from inflow end 306. Accordingly, alignment marker 1202 is radially offset clockwise from the respective commissure post 340. By knowing the radial offset from the respective commissure post 340 as well as the longitudinal offset from inflow end 306, the physician can radially and longitudinally align valve prosthesis 300 during delivery.

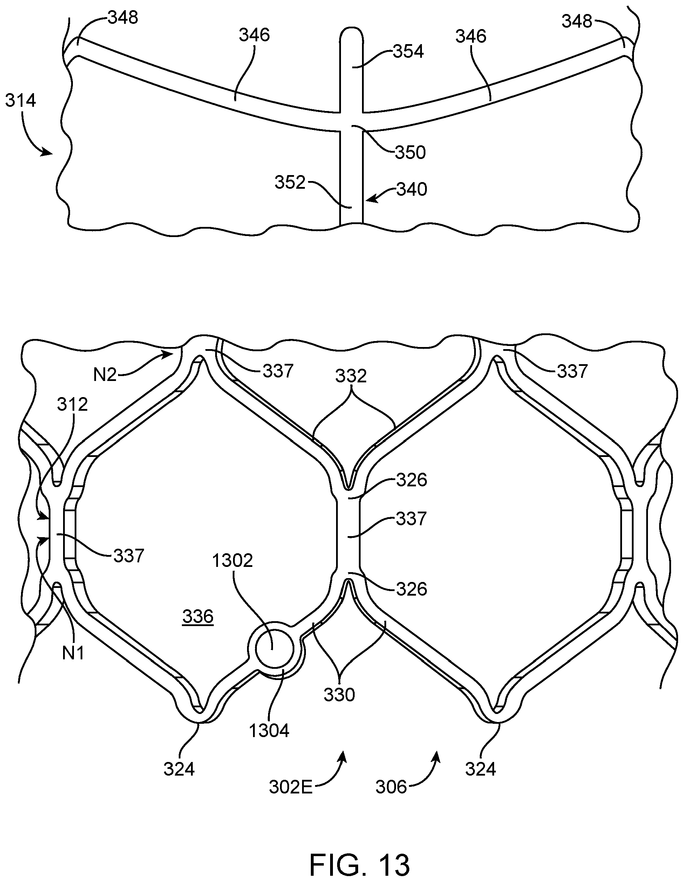

FIG. 13 is a cutaway partial flat plan view of a stent structure 302E for use in valve prosthesis 300 of FIGS. 3, 6 in accordance with one embodiment. Stent structure 302E of FIG. 13 is similar to stent structure 302A of FIG. 8 and only the significant differences are discussed below.

Referring now to FIGS. 8 and 13 together, in place of alignment markers 702, stent structure 302E includes alignment markers 1302 formed in inflow struts 330 of inflow portion 312. More particularly, alignment markers 1302 are formed in inflow struts 330 extending proximally from first row N1 of central nodes 337 aligned with commissure posts 340. In other words, from a central node 337 in first row N1 of central nodes 337 that is aligned with a commissure post 340, an inflow struts 330 extends to an inflow crown 324. In this inflow strut 330, an alignment marker 1302 and containment member 1304 are formed at the center of the inflow strut 330. Alignment marker 1302 and containment member 1304 are similar to alignment marker 702 and containment member 704 and so are not discussed in detail for clarity.

The inflow strut 330 extends to the left in the view of FIG. 13 from the radial position of the respective commissure post 340, or counterclockwise when viewed from inflow end 306. Accordingly, alignment marker 1302 is radially offset counterclockwise from the respective commissure post 340. By knowing the radial offset from the respective commissure post 340 as well as the longitudinal offset from inflow end 306, the physician can radially and longitudinally align valve prosthesis 300 during delivery.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Further disclosed herein is the subject-matter of the following clauses:
1. A valve prosthesis comprising:
   a stent structure comprising:
      an inflow portion comprising:
      a plurality of rows of struts and crowns;
      a plurality of rows of nodes where the crowns of adjacent rows of the plurality of rows of struts and crowns connect, the plurality of rows of nodes comprising a first row of nodes, the first row of nodes being a most proximal row of the plurality of rows of nodes;
   alignment markers disposed at each node of the first row of nodes; and
   a prosthetic valve coupled to the stent structure.
2. The valve prosthesis of Clause 1 wherein the alignment markers comprise radiopaque material.
3. The valve prosthesis of Clause 1 or of any of the preceding Clauses wherein the stent structure further comprises containment members at each node of the first row.
4. The valve prosthesis of Clause 3 wherein the alignment markers are disposed within the containment members.
5. The valve prosthesis of Clause 1 or of any of the preceding Clauses wherein the stent structure further comprises an outflow portion, the outflow portion comprising commissure posts and non-commissure posts, the non-commissure posts being in a shape of a figure 8.
6. The valve prosthesis of Clause 5 wherein the alignment markers are depth of implant markers and the non-commissure posts are clocking markers configured for rotational alignment of the valve prosthesis.
7. The valve prosthesis of Clause 1 or of any of the preceding Clauses wherein the prosthetic valve is a one-piece molded structure.
8. A valve prosthesis comprising:
   a stent structure comprising:
      an outflow portion comprising:
         commissure posts; and
         non-commissure posts; and
      an inflow portion comprising:
         a plurality of rows of struts and crowns;
         a plurality of rows of nodes where the crowns of adjacent rows of struts and crowns connect, the plurality of rows of nodes comprising a first row of nodes, the first row of nodes being a most proximal row of the plurality of rows of nodes;
   alignment markers only at each node of the first row of nodes that is radially aligned with a commissure post; and
   a prosthetic valve coupled to the stent structure.
9. The valve prosthesis of Clause 8 wherein in the first row of nodes, there are three nodes without alignment markers for each node that has an alignment marker.
10. The valve prosthesis of Clause 8 or of any of Clauses 8-9 wherein the alignment markers are depth of implant and clocking markers.
11. The valve prosthesis of Clause 8 or of any of Clauses 8-10 wherein the stent structure further comprises containment members, the alignment markers being disposed within the containment members.
12. The valve prosthesis of Clause 11 further comprising strain relief features at intersections of the containment members and the crowns at the nodes with containment members.
13. The valve prosthesis of Clause 12 wherein the strain relief features comprise cutouts.
14. The valve prosthesis of Clause 12 or of any of Clauses 12-13 wherein the containment members are circles and the crowns are curves, the curves of the crowns abutting the circles of the containment members without any overlap of the curves and the circles to define the strain relief features.
15. A valve prosthesis comprising:
   a stent structure comprising:
      an outflow portion comprising:
         commissure posts; and
         non-commissure posts; and
      an inflow portion comprising:
         a plurality of rows of struts and crowns;
         a plurality of rows of nodes where the crowns of adjacent rows of struts and crowns connect;
   an alignment marker disposed in a first strut extending from a first node of nodes of the plurality of rows of nodes, the first node being radially aligned with a commissure post; and
   a prosthetic valve coupled to the stent structure.
16. The valve prosthesis of Clause 15 wherein the first strut extends distally from the first node.
17. The valve prosthesis of Clause 15 or of any of Clauses 15-16 wherein the first strut extends proximally from the first node.
18. The valve prosthesis of Clause 15 or of any of Clauses 15-17 wherein the alignment marker comprises a plurality of alignment markers, wherein the plurality of alignment markers are disposed formed in struts extending from nodes radially aligned with the commissure posts.
19. The valve prosthesis of Clause 18 wherein the alignment markers are depth of implant and clocking markers.
20. The valve prosthesis of Clause 15 or of any of Clauses 15-19, wherein the prosthetic valve includes commissures, and wherein the commissures are coupled to the commissure posts of the stent structure.

## Claims

1. A valve prosthesis comprising:
a stent structure comprising:
an inflow portion comprising:
a plurality of rows of struts and crowns;
a plurality of rows of nodes where the crowns of adjacent rows of the plurality of rows of struts and crowns connect, the plurality of rows of nodes comprising a first row of nodes, the first row of nodes being a most proximal row of the plurality of rows of nodes;
alignment markers disposed at each node of the first row of nodes; and
a prosthetic valve coupled to the stent structure.

2. The valve prosthesis of Claim 1 wherein the alignment markers comprise radiopaque material.

3. The valve prosthesis of any of the preceding Claims wherein the stent structure further comprises containment members at each node of the first row, wherein optionally the alignment markers are disposed within the containment members.

4. The valve prosthesis of any of the preceding Claims wherein the stent structure further comprises an outflow portion, the outflow portion comprising commissure posts and non-commissure posts, the non-commissure posts being in a shape of a figure 8, wherein optionally the alignment markers are depth of implant markers and the non-commissure posts are clocking markers configured for rotational alignment of the valve prosthesis.

5. The valve prosthesis of any of the preceding Claims wherein the prosthetic valve is a one-piece molded structure.

6. A valve prosthesis comprising:
a stent structure comprising:
an outflow portion comprising:
commissure posts; and
non-commissure posts; and
an inflow portion comprising:
a plurality of rows of struts and crowns;
a plurality of rows of nodes where the crowns of adjacent rows of struts and crowns connect, the plurality of rows of nodes comprising a first row of nodes, the first row of nodes being a most proximal row of the plurality of rows of nodes;
alignment markers only at each node of the first row of nodes that is radially aligned with a commissure post; and
a prosthetic valve coupled to the stent structure.

7. The valve prosthesis of Claim 6 wherein in the first row of nodes, there are three nodes without alignment markers for each node that has an alignment marker, and/or wherein the alignment markers are depth of implant and clocking markers.

8. The valve prosthesis of any of Claims 6-7 wherein the stent structure further comprises containment members, the alignment markers being disposed within the containment members.

9. The valve prosthesis of Claim 8 further comprising strain relief features at intersections of the containment members and the crowns at the nodes with containment members.

10. The valve prosthesis of Claim 9 wherein the strain relief features comprise cutouts, and/or wherein the containment members are circles and the crowns are curves, the curves of the crowns abutting the circles of the containment members without any overlap of the curves and the circles to define the strain relief features.

11. A valve prosthesis comprising:
a stent structure comprising:
an outflow portion comprising:
commissure posts; and
non-commissure posts; and
an inflow portion comprising:
a plurality of rows of struts and crowns;
a plurality of rows of nodes where the crowns of adjacent rows of struts and crowns connect;
an alignment marker disposed in a first strut extending from a first node of nodes of the plurality of rows of nodes, the first node being radially aligned with a commissure post; and
a prosthetic valve coupled to the stent structure.

12. The valve prosthesis of Claim 11 wherein the first strut extends distally from the first node.

13. The valve prosthesis of any of Claims 11-12 wherein the first strut extends proximally from the first node.

14. The valve prosthesis of any of Claims 11-13 wherein the alignment marker comprises a plurality of alignment markers, wherein the plurality of alignment markers are disposed formed in struts extending from nodes radially aligned with the commissure posts, wherein optionally the alignment markers are depth of implant and clocking markers.

15. The valve prosthesis of any of Claims 11-14, wherein the prosthetic valve includes commissures, and wherein the commissures are coupled to the commissure posts of the stent structure.
